**Europäisches Patentamt**

**European Patent Office**

**Office européen des brevets**

(11) Veröffentlichungsnummer: **0 102 561**
**B1**

(12) # EUROPÄISCHE PATENTSCHRIFT

(45) Veröffentlichungstag der Patentschrift:
**10.04.85**

(51) Int. Cl.⁴: **C 07 C 51/56,** C 07 C 53/12

(21) Anmeldenummer: **83107993.4**

(22) Anmeldetag: **12.08.83**

(54) **Verfahren zur Herstellung von Essigsäureanhydrid.**

(30) Priorität: **28.08.82 DE 3232066**

(43) Veröffentlichungstag der Anmeldung:
**14.03.84 Patentblatt 84/11**

(45) Bekanntmachung des Hinweises auf die Patenterteilung:
**10.04.85 Patentblatt 85/15**

(84) Benannte Vertragsstaaten:
**BE DE FR GB IT NL**

(56) Entgegenhaltungen:
**DE - A - 2 658 216**

(73) Patentinhaber: **HOECHST AKTIENGESELLSCHAFT,
Postfach 80 03 20, D-6230 Frankfurt am Main 80 (DE)**

(72) Erfinder: **Vogt, Wilhelm, Dr., Bellerstrasse 74,
D-5030 Hürth (DE)**
Erfinder: **Jägers, Erhard, Dr., Schwarzwaldstrasse 1,
D-5303 Bornheim (DE)**
Erfinder: *Glaser, Hermann, Magdalenenweg 16,
D-5042 Erftstadt (DE)*

## Beschreibung

Die Erfindung betrifft ein Verfahren zur Herstellung von Essigsäureanhydrid durch Umsetzung von Methylacetat und/oder Dimethylether mit Kohlenmonoxid, ggf. in Mischungen mit bis zu 30 Vol.-% Wasserstoff, unter praktisch wasserfreien Bedingungen bei Temperaturen von 390 bis 540 K und Drücken von 1 bis 300 bar in Gegenwart eines Katalysatorsystems aus Nickel oder Nickelverbindungen, Jod und/oder dessen Verbindungen sowie einer tertiären oder quaternären organischen Stickstoff-, Phosphor-, Arsen- oder Antimonverbindung.

Ein derartiges Verfahren kann bereits der US-PS Nr. 2729651 entnommen werden. Während dort überwiegend Nickelkomplexe wie z.B. Triphenylethylphosphoniumnickeltetrajodid eingesetzt werden, kann auch von z.B. Tetramethylammoniumjodid und Nickeljodid oder von Nickelpulver, Jod, Triethylamin und Ethyljodid ausgegangen werden. Obwohl Drücke bis zu 700 bar angewendet werden, sind die Raum/Zeit-Leistungen an Essigsäureanhydrid nur gering. Die Umsetzungszeiten liegen zwischen 5 und 26 h. Sowohl der hohe Druck als auch die geringen Leistungen machen ein Arbeiten nach diesem Verfahren unwirtschaftlich.

Da das Reaktionsmedium korrosiv ist, so dass der Autoklav aus einer Legierung von Hastelloy B oder C oder aus Tantal hergestellt werden muss, bedürfen derartige Carbonylierungsverfahren eines sehr hohen Investitionsaufwandes.

Die vorliegende Erfindung ist nun dadurch gekennzeichnet, dass das Katalysatorsystem zusätzlich Vanadium oder Niob oder deren Verbindungen enthält.

Dadurch wird überraschenderweise eine Aktivierung des in der US-PS Nr. 2729651 beschriebenen Katalysatorsystems erreicht, wodurch erheblich höhere Raum/Zeit-Leistungen erhalten werden, was wiederum die Wirtschaftlichkeit des Verfahrens entscheidend verbessert. Mit dem Verfahren der Erfindung werden bei 460 K Leistungen erzielt, die mit denen des ungleich teureren Rhodiumkatalysatorsystems vergleichbar sind. Eine Bildung von Ethylidendiacetat wird trotz der Anwesenheit von Wasserstoff nicht beobachtet.

Als Nickelverbindungen kann man z.B. Nickelcarbonyl, -acetonat, -halogenid, -acetat, -sulfat oder -cyanid einsetzen.

Vanadium oder Niob können als Metall oder als Verbindungen in jeder Oxidationsstufe, z.B. als Chloride ($VCl_3$, $NbCl_5$), Vanadyl(V)alkylat oder als Acetylacetonate ($VO[C_5H_7O_2]_2$) eingesetzt werden. Das Molverhältnis von Ni/V oder von Ni/Nb ist bevorzugt 1 : (0,1-4).

Als Jodverbindung wird bevorzugt Methyljodid oder Jodwasserstoff verwendet. Als tertiäre Organostickstoff- oder Organophosphorverbindungen kommen Amine, Phosphine oder Aminophosphine in Frage, z.B. Trialkylamine, N,N-Dialkylanilin, Pyridin, N-Methylimidazol, 3-Picolin, 2,4-Lutidin, 3,4-Lutidin, Chinolin, Trialkylphosphine wie Tributylphosphin, Trioctylphosphin, Trilaurylphosphin oder Triarylphosphine wie Triphenylphosphin. Die Organostickstoff- oder Organophosphorverbindungen können jedoch auch quaternisiert mit Methylhalogenid oder Halogenwasserstoff eingesetzt werden, z.B. als N-Methylpyridiniumhalogenid, N,N-Dimethylimidazoliumhalogenid, N-Methyl-3-picoliniumhalogenid, N-Methyl-2,4-lutidiniumhalogenid, N-Methyl-3,4-lutidiniumhalogenid, N-Methylchinoliniumhalogenid, Tributylmethylphosphoniumhalogenid, Trioctylmethylphosphoniumhalogenid, Trilaurylmethylphosphoniumhalogenid, Triphenylmethylphosphoniumhalogenid, wobei unter Halogenid jeweils Chlorid, Bromid oder vorzugsweise Jodid zu verstehen sind. Als organische Arsen- und Antimonverbindungen können Arsine und Stibine mit Vorteil verwendet werden. Insgesamt gesehen werden die Organophosphorverbindungen jedoch bevorzugt.

Die einzelnen Reaktionskomponenten, d.h. Methylacetat oder Dimethylether/Nickel(-Verbindung)/Jod(-Verbindung)/Stickstoff-, Phosphor-, Arsen- oder Antimonverbindung/Vanadium(-Verbindung) oder Niob(-Verbindung), werden bevorzugt im Molverhältnis 1 : (0,001-0,1) : (0,01-1) : (0,01-1) : (0,0005-0,1) eingesetzt.

Die erfindungsgemässe Carbonylierungsreaktion kann auch in Gegenwart von Lösemitteln durchgeführt werden. Als solche sind Essigsäure oder Amide wie N-Methylpyrrolidon, N,N-Diethylacetamid oder schwefelhaltige Lösemittel wie Sulfolan besonders geeignet.

Da man gemäss der Erfindung bei wesentlich geringeren Drucken als beim Verfahren der US-PS Nr. 2729651, bevorzugt bei 40 bis 150 bar, arbeiten kann, sind die Investitionskosten für Druckapparaturen deutlich geringer.

*Beispiel 1:* (Vergleichsbeispiel)

250 g Methylacetat, 50 g Methyljodid, 102 g Methyltributylphosphoniumjodid und 7,66 g Nickel(II)acetylacetonat werden in einem Hastelloy-Autoklaven mit 100 bar Kohlenmonoxid bei 460 K umgesetzt. Nach einer Reaktionszeit von 265 min findet man 172,6 g Acetanhydrid, entsprechend einer Leistung von 106,4 g $Ac_2O$ je Liter Reaktionslösung und Stunde.

*Beispiel 2:* (Vergleichsbeispiel)

250 g Methylacetat, 50 g Methyljodid, 102 g Methyltributylphosphoniumjodid und 7,66 g Nickel(II)acetylacetonat werden mit 100 bar $CH/H_2$ im Vol.-Verhältnis 10:1 bei 460 K umgesetzt. Nach einer Reaktionszeit von 188 min findet man 165 g Acetanhydrid, entsprechend 142 g $Ac_2O$ je Liter Reaktionslösung und Stunde.

*Beispiel 3:*

250 g Methylacetat, 50 g Methyljodid, 102 g Methyltributylphosphoniumjodid, 7,66 g Nickel(II)acetylacetonat und 9,44 g Vanadium(III)chlorid werden bei 460 K mit 100 bar CO umgesetzt. Nach 200 min Reaktionszeit findet man 185 g Acetanhydrid, entsprechend 149 g $Ac_2O$ je Liter Reaktionslösung und Stunde.

*Beispiel 4:*

250 g (3,38 mol) Methylacetat, 50 g (0,352 mol) Methyljodid, 102 g (0,296 mol) Methyltributylphosphoniumjodid, 7,66 g (0,03 mol) Nickel(II)acetylacetonat und 7,3 g (0,03 mol) Vanadyl(V)-n-propylat $(VO[OC_3H_7]_3)$ werden mit 100 bar $CO/H_2$ im Vol.-Verhältnis 10:1 bei 460 K umgesetzt. Nach 54 min isoliert man 172 g Acetanhydrid, entsprechend einer Leistung von 515,2 g $Ac_2O$ je Liter Reaktionslösung und Stunde.

*Beispiel 5:*

250 g Methylacetat, 50 g Methyljodid, 102 g Methyltributylphosphoniumjodid, 7,66 g (0,03 mol) Nickel(II)acetylacetonat und 9,44 g (0,06 mol) Vanadium(III)chlorid werden bei 475 K mit 100 bar $CO/H_2$ im Vol.-Verhältnis 10:1 zur Reaktion gebracht. Nach 36 min findet man 163 g Acetanhydrid, entsprechend 732,4 g $Ac_2O$ je Liter Reaktionslösung und Stunde.

*Beispiel 6:*

250 g Methylacetat, 50 g Methyljodid, 102 g Methyltributylphosphoniumjodid, 7,66 g (0,03 mol) Nickel(II)acetylacetonat und 16,2 g (0,06 mol) Niob(V)chlorid werden bei 460 K mit 100 bar $CO/H_2$ im Vol.-Verhältnis 10:1 umgesetzt. Nach 94 min findet man 159 g Acetanhydrid, entsprechend 274 g $Ac_2O$ je Liter Reaktionslösung und Stunde.

*Beispiel 7:*

250 g Methylacetat, 50 g Methyljodid, 102 g Methyltributylphosphoniumjodid, 5,12 g Nickeltetracarbonyl (0,03 mol) und 7,92 g Vanadyl(IV)acetylacetonat (0,03 mol) werden bei 460 K mit 100 bar $CO/H_2$ im Vol.-Verhältnis 10:1 umgesetzt. Nach 35 min findet man 179,9 g Acetanhydrid, entsprechend 834 g $Ac_2O$ je Liter Reaktionslösung und Stunde.

### Patentansprüche

1. Verfahren zur Herstellung von Essigsäureanhydrid durch Umsetzung von Methylacetat und/oder Dimethylether mit Kohlenmonoxid, ggf. in Mischungen mit bis zu 30 Vol.-% Wasserstoff, unter praktisch wasserfreien Bedingungen bei Temperaturen von 390 bis 540 K und Drucken von 1 bis 300 bar in Gegenwart eines Katalysatorsystems aus Nickel oder Nickelverbindungen, Jod und/oder dessen Verbindungen sowie einer tertiären oder quaternären organischen Stickstoff-, Phosphor-, Arsen- oder Antimonverbindung, dadurch gekennzeichnet, dass das Katalysatorsystem zusätzlich Vanadium oder Niob oder deren Verbindungen enthält.

2. Verfahren nach Anspruch 1, dadurch gekennzeichnet, dass man Methylacetat oder Dimethylether/Nickel(-Verbindung)/Jod(-Verbindung)/Stickstoff-, Phosphor-, Arsen- oder Antimonverbindung/Vanadium(-Verbindung) oder Niob(-Verbindung) im Molverhältnis 1:(0,001-0,1):(0,01-1):(0,01-1):(0,0005-0,1) einsetzt.

### Claims

1. Process for making acetic anhydride by reacting methyl acetate and/or dimethylether with carbon monoxide, optionally in admixture with up to 30 vol.-% hydrogen, under practically anhydrous conditions at temperatures of 390 to 540 K and under pressures of 1 to 300 bar in the presence of a catalyst system containing nickel or nickel compounds, iodine and/or its compounds as well as a tertiary or quaternary organic nitrogen, phosphorus, arsenic or antimony compound, characterized in that the catalyst system additionally contains vanadium or niobium or their compounds.

2. Process as claimed in Claim 1, wherein methyl acetate or dimethylether/nickel(-compound)/iodine(-compound)/nitrogen, phosphorus, arsenic or antimony compound/vanadium (-compound) or niobium(-compound) are used in a molar ratio of 1:(0.001-0.1):(0.01-1): (0.01-1):(0.0005-0.1).

### Revendications

1. Procédé de préparation de l'anhydride acétique par réaction d'acétate de méthyle et/ou d'éther diméthylique avec le monoxyde de carbone, éventuellement en association avec jusqu'à 30% en volume d'hydrogène, dans des conditions pratiquement anhydres à des températures de 390 à 540 K et sous des pressions de 1 à 300 bar en présence d'un système catalytique constitué par du nickel ou des composés de nickel, de l'iode et/ou ses composés, ainsi qu'un composé organique d'azote, de phosphore, d'arsenic ou d'antimoine tertiaire ou quaternaire, caractérisé en ce que le système catalytique contient en outre du vanadium ou du niobium ou leurs composés.

2. Procédé selon la revendication 1, caractérisé en ce que l'on utilise l'acétate de méthyle ou l'éther diméthylique, le (composé de) nickel, l'iode (le composé d'iode), le composé d'azote, de phosphore, d'arsenic ou d'antimoine, le (composé de) vanadium ou le (composé de) niobium dans des proportions molaires de 1:(0,001-01):(0,01-1):(0,01-1):(0,0005-0,1).